# EUROPEAN PATENT SPECIFICATION

(11) **EP 4 547 175 B1**
(45) Date of publication and mention of the grant of the patent: **29.07.2026**
(21) Application number: 23748612.1
(22) Date of filing: 21.06.2023
(51) Int. Cl.: A61F 13/47, A61F 13/475, A61F 13/539

(54) **DISPOSABLE ABSORBENT INSERT AND ITS MANUFACTURING PROCESS**
SAUGFÄHIGE EINWEGMATTE UND ENTSPRECHENDES HERSTELLUNGSVERFAHREN
TAPIS ABSORBANT JETABLE ET PROCÉDÉ DE FABRICATION ASSOCIÉ

(30) Priority: 29.06.2022 IT 202200013720
(43) Date of publication of application: 07.05.2025
(73) Proprietor: Teknoweb Converting Srl, 26020 Palazzo Pignano (CR) (IT)
(72) Inventor: MANDOTTI, Pierangelo, 26020 PALAZZO PIGNANO (CR) (IT)
(74) Representative: Mari, Marco Giovanni
(86) International application number: PCT/IT2023/050147
(87) International publication number: WO 2024/003955

(56) References cited:
- US-A1- 2006 069 368

## Description

### Technical field of application

The present invention relates to the field of sanitary devices and in particular it relates to a disposable absorbent mat, particularly of the type for infants or adults but usable in general to protect any surface which may potentially come into contact with urine or other type of organic liquid, even of animals.

The invention also relates to a process for manufacturing said disposable absorbent mat.

### Background art

Traditional disposable absorbent mats have a quadrangular shape and substantially comprise a sheet of liquid-impermeable material and a sheet of liquid-permeable material, coupled together, with an absorbent pad interposed therein, which forms the core of the mat.

Said sheet of liquid-impermeable material is generally a sheet of polyethylene, and has the task of stopping the liquid which would tend to flow downwards by gravity.

Said sheet of liquid-permeable material is usually made of a hydrophilic non-woven fabric, and has the task of letting the liquids pass, avoiding the onset of allergic reactions on the user's skin.

Said absorbent pad comprises cellulose, technically called FLUFF, optionally mixed with granules of superabsorbent material, technically called SAP, or polymers.

The cellulose is enclosed between two layers of cellulose tissue glued together and is dosed in such a way as to guarantee maximum absorbency to the mat, compatibly with the final cost of the product.

All the layers that make up the mat are joined together by gluing or welding.

In traditional absorbent mats, the sheet of impermeable material and the sheet of permeable material have the same dimensions, while the absorbent pad has smaller dimensions so as to be entirely contained inside, and so as to leave respective coupling edges.

The seal against lateral leakage of liquids is therefore guaranteed only by the reciprocal bonding of these edges.

Disadvantageously, the risk of leakage of liquids passing from the absorbent pad through the edges of the only superimposed and glued sheets is in any case high. Liquid leaks are therefore possible along all four sides of the mat.

Other examples of known absorbent mats provide, in order to solve this drawback at least in part, that at least one pair of sides is closed by turning over a pair of edges of the sheet of liquid-impermeable material on the area occupied by the absorbent pad, below the sheet of liquid-permeable material.

The absorbent mat thus formed in fact comprises lateral closing means against the leakage of liquids affecting two of its four sides.

Although improving, said absorbent mats still have sealing limits to the leakage of liquids along two sides of the same.

On the other hand, it would also be impossible to turn over the other pair of edges which, due to the in-line production process of the mats, are arranged orthogonally to the direction of advancement of the product being manufactured.

### Disclosure of the invention

The object of the present invention is to eliminate the aforementioned drawbacks and disadvantages.

The object of the invention is therefore that of providing a perfectly sealed absorbent mat, which totally prevents the lateral leakage of the liquids absorbed by the pad.

It is also an object of the invention to provide an absorbent mat which can be easily produced, by means of a fast and cost-effective manufacturing process.

The objects are achieved with a disposable absorbent mat, substantially quadrangular in shape, comprising:
- a liquid absorbing pad having a predefined length and width delimiting an effective area;
- a sheet of liquid-impermeable material, having a predefined length and width greater than the length and width of said absorbent pad, coupled laid under said absorbent pad, so as to define a first and a second pair of opposite free edges, arranged outside the effective area occupied by said absorbent pad;
- a sheet of liquid-permeable material, coupled superimposed on said absorbent pad on the opposite side with respect to said sheet of liquid-impermeable material,
wherein said first pair of free edges of said sheet of liquid-impermeable material is turned up on said effective area occupied by said absorbent pad to define first lateral closing means of said absorbent pad against the leakage of liquids,
characterized in that said sheet of liquid-permeable material has a predefined length and width equal to the length and width of said absorbent pad, and in that said absorbent mat comprises a first and a second strip of liquid-impermeable material placed respectively straddling each free edge of said second pair of free edges and said sheet of liquid-permeable material, wherein said first and second strips are coupled at least to said second pair of free edges to define second lateral closing means of said absorbent pad against the leakage of liquids, arranged orthogonally with respect to said first lateral closing means.

Advantageously, said first pair of free edges of said sheet of liquid-impermeable material is turned up on said liquid-permeable material.

Even more advantageously, said first and second strips are superimposed on the ends of said first lateral closing means formed by the first pair of free edges which have been turned up.

According to possible variants of the invention:
- said first and second strips of impermeable material comprise a hydrophobic non-woven fabric;
- said sheet of liquid-impermeable material comprises polyethylene;
- said sheet of liquid-permeable material comprises hydrophilic non-woven fabric;
said absorbent pad is composite, and comprises at least one tissue made of hydrophilic material and a layer of absorbent cellulose, wherein said at least one tissue faces said sheet of liquid-impermeable material and said absorbent cellulose layer faces said sheet of liquid-permeable material.

According to further aspects of the invention, said absorbent pad, said sheet of liquid-impermeable material, said sheet of liquid-permeable material, said pairs of edges and said strips are coupled together by glue or welding.

The invention also relates to a process for manufacturing a disposable absorbent mat as described above, characterized in that it comprises the steps of:
- providing a web of liquid-impermeable material having a predefined width and unwinding it according to a direction of advancement;
- providing a plurality of liquid absorbing pads, each having a predefined length and width, said width being less than the width of said web;
- providing a plurality of sheets of liquid-permeable material, each having a predefined length and width equal to the length and width of said absorbent pads;
- coupling together said absorbent pads and said sheets of liquid-permeable material to create multilayer absorbent panels;
- applying said multilayer absorbent panels on top of said web of liquid-impermeable material according to said direction of advancement, in sequence and spaced apart, so as to define, along said web, effective areas and a first pair of opposite free edges along said direction of advancement and transverse free areas with respect to said direction of advancement between each multilayer absorbent panel;
- turning up said first pair of free edges of said web of liquid-impermeable material on said effective areas occupied by said multilayer absorbent panels to define first lateral closing means of said absorbent mat against the leakage of liquids;
- providing strips of a liquid-impermeable material;
- applying said strips straddling at least a portion of said free transverse areas and said effective areas occupied by said multilayer absorbent panels, to obtain second lateral closing means against the leakage of liquids, arranged orthogonally to said first lateral closing means;
- transversely cutting said web to obtain said disposable absorbent mat.

In a preferred variant, said process further comprises the steps of:
- providing alternative strips of a liquid-impermeable material having a width greater than the width of said free transverse areas along said direction of advancement of said web;
- applying each of said alternative strips between two consecutive multilayer absorbent panels;
- transversely cutting said web at a median line of said alternative strips to obtain said strips.

Alternatively, said steps of coupling together said absorbent pads and said sheets of liquid-permeable material, of applying said multilayer absorbent panels on top of said web of liquid-impermeable material, and of applying said strips, comprise the steps of gluing or welding said components together.

The main advantage of the invention consists in the fact that the absorbent mat is closed, liquid-tight, along all four sides.

In addition to the turned-up edges of two parallel sides of the mat, the sides at orthogonal thereto are also advantageously sealed so that any liquids not absorbed by the pad cannot escape.

Closing the mat on four sides also allows for the use of simplified absorbent pads, i.e. also made only with a basic tissue paper and the free cellulose layer on top: the sheet of permeable material, sized like the pad and coupled to it, will act as second top tissue.

By being able to save on a tissue layer in the construction of the absorbent pad, there is a substantial reduction in production costs.

Furthermore, in the in-line manufacturing process as claimed, it is possible to use a single strip of impermeable material to close two consecutive mats: in fact, it is possible to apply a single strip between two consecutive multilayer absorbent panels, occupying an entire free area and two effective areas occupied by two adjacent multilayer absorbent panels, and then cutting said strip, and the underlying web of impermeable material, along a median line, to obtain the individual mats. The saving of material for the number of strips actually used is evident.

### Brief description of the drawings

These and other advantages of shall appear more clearly from the following description of a preferred embodiment of the invention, made by way of an indicative and non-limiting example with reference to the figures, in which:
Figs. 1, 2, 3 show, respectively in plan view from above, and in sections, partially exploded and with proportions deliberately deformed for clarity of representation, along two vertical planes perpendicular to each other, an absorbent mat according to the invention;
Fig. 4 shows, schematically, steps of a manufacturing process of the absorbent mat of Figure 1 according to a possible preferred variant of the invention.

### Detailed description of preferred embodiments of the invention

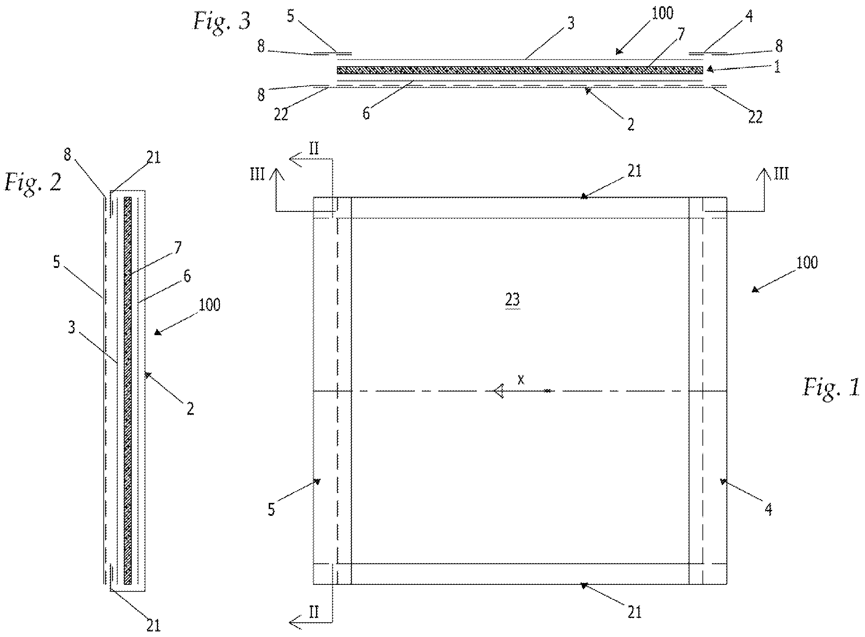

With reference to Figs. 1-3, the invention relates to a disposable absorbent mat, substantially quadrangular in shape.

Said absorbent crosspiece 100, of the disposable type, is stratified and comprises:
- a liquid absorbent pad 1;
- a sheet of liquid-impermeable material 2 coupled laid under said absorbent pad 1;
- a sheet of liquid-permeable material 3, coupled superimposed on said absorbent pad 1 on the opposite side with respect to said sheet of liquid-impermeable material 2.

Said sheet of liquid-impermeable material 2 comprises polyethylene.

Said sheet of liquid-permeable material 3 comprises a loosely woven hydrophilic non-woven fabric.

Said absorbent pad 1 is composite, and comprises a tissue paper 6 in hydrophilic material and a layer of absorbent cellulose 7, where inside said absorbent mat 100, said tissue paper 6 faces said sheet of liquid-impermeable material 2 and said layer of absorbent cellulose 7 faces towards said sheet of liquid-permeable material 3.

Compared to the traditional absorbent pads made up of two layers of tissue which hold the cellulose layer between them, the pad which can be used in the mat of the invention can advantageously do without a layer of tissue paper, this being in fact replaced, in its function, by the sheet of hydrophilic non-woven fabric coupled to the pad.

Absorbent pads can also be obtained with sheets of non-woven fabric, as an alternative to tissue paper, and with layers of cellulose mixed with super-absorbent granules or polymers.

Said absorbent pad 1 has a predefined length and width delimiting an effective area; said sheet of liquid-permeable material 3 has a predefined length and width equal to the length and width of said absorbent pad 1; said sheet of liquid-impermeable material 2 has a predefined length and width greater than the length and width of said absorbent pad 1, so as to define a first 21 and a second 22 pair of free edges external to said effective area 23, i.e. the area effectively absorbent, occupied by said absorbent pad 1.

With particular reference to the section of Fig. 2, first lateral closing means of said absorbent mat 100 against the leakage of liquids are illustrated.

A first pair of free edges 21 of said sheet of liquid-impermeable material 2 is turned up on said effective area 23 represented by said absorbent pad 1 to precisely define said first lateral closing means.

With particular reference to the section of Fig. 3, on the other hand, second lateral closing means of said absorbent mat 100 against the leakage of liquids are illustrated, arranged orthogonally to said first lateral closing means.

Said absorbent mat 100 comprises a first 4 and a second 5 strip of liquid-impermeable material placed respectively astride said second pair of free edges 22, orthogonal to said first pair of free edges 21, and said sheet of liquid-permeable material 3.

In particular, said first 4 and second 5 strips are coupled superimposed on at least said second pair of free edges 22, to define said second lateral closing means of said absorbent pad 1 and in particular they are superimposed on the ends of said first lateral closing means formed by the first pair of free edges 21 which have been turned up.

Said first 4 and second 5 strips of impermeable material comprise a hydrophobic non-woven fabric or, advantageously, the same material which makes the liquid-impermeable sheet 2 of the absorbent mat 100 placed under the absorbent pad 1, i.e. polyethylene.

All the layers of the illustrated absorbent mat 100, i.e. said absorbent pad 1, said sheet of liquid-impermeable material 2, said sheet of liquid-permeable material 3, said pairs of free edges 21, 22 and said strips 4, 5, are coupled to each other by means of a layer of glue 8.

Alternatively, said layers may be joined together by welding technique of the known type.

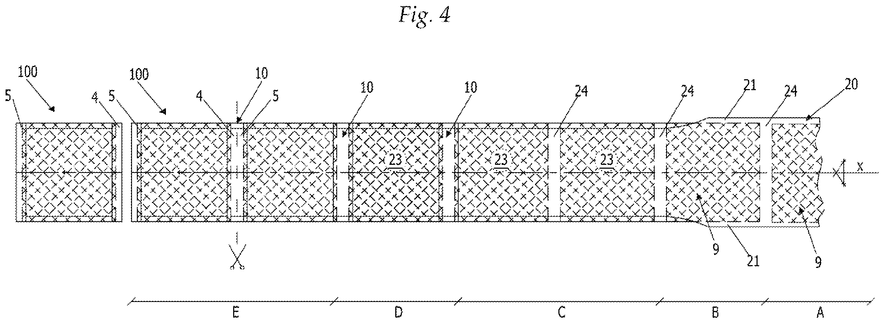

The diagram of Fig. 4 illustrates part of the manufacturing process of an absorbent mat 100 as described above.

The first step (section A) provides for unwinding, according to an advancement direction x, a web 20 of liquid-impermeable material, for example polyethylene, having a certain predefined width.

Parallel to the web unwinding line, 9 multilayer absorbent panels are prepared.

For the assembly of the multilayer absorbent panels 9, the following steps are followed (not illustrated):
- providing a plurality of liquid absorbing pads 1, each having a predefined length and width, said width being less than the width of said web 20;
- providing a plurality of sheets of liquid-permeable material 3, each having a predefined length and width equal to the length and width of said absorbent pads 1;
- coupling together, by gluing or welding, said absorbent pads 1 and said sheets of liquid-permeable material 3 to create said multilayer absorbent panels 9.

Next, again with reference to the diagram in Fig. 4, said multilayer absorbent panels 9 are applied above said web 20 of liquid-impermeable material, along said direction of advancement x, centrally to said web, in sequence and spaced apart from each other (section B).

Said multilayer absorbent panels 9 are positioned on said web 20 in such a way as to define effective absorption areas 23 and in such a way as to define, along said web 20, a first pair of free edges 21 opposite each other along said direction of advancement x and free transverse areas 24 with respect to said direction of advancement x between each multilayer absorbent panel 9.

The subsequent steps concern the construction of first lateral closing means of said absorbent mat 100 against the leakage of liquids (section C).

These operations in fact comprise the step of turning up said first pair of free edges 21 of said web 20 of liquid-impermeable material on said effective areas 23 occupied by said multilayer absorbent panels 9 in a sequence.

With reference to sections D and E of the diagram in Fig. 4, the steps for making second lateral closing means of said absorbent mat 100 against the leakage of liquids are illustrated, arranged orthogonally to said first lateral closing means are illustrated, i.e. the steps of:
- providing alternative strips 10 of a liquid-impermeable material;
- applying said alternative strips 10 between said transversal free areas 24 and said effective areas 23 occupied by said multilayer absorbent panels 9 (section D)
- transversely cutting said web 20 to obtain said disposable absorbent mats 100 provided with said second lateral closing means (section E).

In particular, said alternative strips 10 of liquid-impermeable material have a width greater than the width of said transversal free areas 24 measured along said direction of advancement x of said web 20, so that each strip 10 entirely occupies a transversal free area 24 until two consecutive 23 effective areas are overlapped.

The transversal cut of said strip 20, at a median line of said strips 10, then separates the mats 100 and divides the strips 10 each into the two strips 4, 5 which form said second lateral closing means.

Alternatively, to make said second lateral closing means, strips 4, 5 of liquid-impermeable material could be used having a width such as to overlap only an effective area 23 and partially said transversal free area 24, so that the cutting step affects just said web 20.

## Claims

1. Disposable absorbent mat (100), substantially quadrangular in shape, comprising:
- a liquid absorbing pad (1) having a predefined length and width delimiting an effective area (23);
- a sheet of liquid-impermeable material (2), having a predefined length and width greater than the length and width of said absorbent pad (1), coupled laid under said absorbent pad (1), so as to define a first (21) and a second (22) pair of opposite free edges, arranged outside the effective area (23) occupied by said absorbent pad (1);
- a sheet of liquid-permeable material (3), coupled superimposed on said absorbent pad (1) on the opposite side with respect to said sheet of liquid-impermeable material (2),
wherein said first pair of free edges (21) of said sheet of liquid-impermeable material (2) is turned up on said effective area (23) occupied by said absorbent pad (1) to define first lateral closing means of said absorbent pad (1) against the leakage of liquids, **characterized in that** said sheet of liquid-permeable material (3) has a predefined length and width equal to the length and width of said absorbent pad (1), and **in that** said absorbent mat (100) comprises a first (4) and a second (5) strip of liquid-impermeable material placed respectively straddling each free edge of said second pair of free edges (22) and said sheet of liquid-permeable material (3), wherein said first (4) and second (5) strips are coupled at least to said second pair of free edges (22) to define second lateral closing means of said absorbent pad (1) against the leakage of liquids, arranged orthogonally with respect to said first lateral closing means.

2. Disposable absorbent mat (100) according to claim 1, wherein said first pair of free edges (21) of said sheet of liquid-impermeable material (2) is turned up on said liquid-permeable material (3).

3. Disposable absorbent mat (100) according to claim 2, wherein said first (4) and second (5) strips are superimposed on the ends of said first lateral closing means formed by the first pair of free edges (21) which have been turned up.

4. Disposable absorbent mat (100) according to any one of the preceding claims, **characterized in that** said first (4) and second (5) strips of impermeable material comprise hydrophobic non-woven fabric.

5. Disposable absorbent mat (100) according to any one of the preceding claims, **characterized in that** said sheet of liquid-impermeable material (2) comprises polyethylene.

6. Disposable absorbent mat (100) according to any one of the preceding claims, **characterized in that** said sheet of liquid-permeable material (3) comprises hydrophilic non-woven fabric.

7. Disposable absorbent mat (100) according to any one of the preceding claims, **characterized in that** said absorbent pad (1) is composite, and comprises at least one tissue (6) made of hydrophilic material and a layer (7) of absorbent cellulose, wherein said at least one tissue (6) faces said sheet of liquid-impermeable material (2) and said absorbent cellulose layer (7) faces said sheet of liquid-permeable material (3).

8. Disposable absorbent mat (100) according to any one of the preceding claims, **characterized in that** said absorbent pad (1), said sheet of liquid-impermeable material (2), said sheet of liquid-permeable material (3), said pairs of edges (21 , 22) and said strips (4, 5) are coupled together by glue (8) or welding.

9. Process for manufacturing a disposable absorbent mat (100) according to any one of claims 1-8, **characterized in that** it comprises the steps of:
- providing a web (20) of liquid-impermeable material having a predefined width and unwinding it according to a direction of advancement (x);
- providing a plurality of liquid absorbing pads (1), each having a predefined length and width, said width being less than the width of said web (20);
- providing a plurality of sheets of liquid-permeable material (3), each having a predefined length and width equal to the length and width of said absorbent pads (1);
- coupling together said absorbent pads (1) and said sheets of liquid-permeable material (3) to create multilayer absorbent panels (9);
- applying said multilayer absorbent panels (9) on top of said web (20) of liquid-impermeable material according to said direction of advancement (x), in sequence and spaced apart, so as to define, along said web (20), effective areas (23) and a first pair of opposite free edges (21) along said direction of advancement (x) and transverse free areas (24) with respect to said direction of advancement (x) between each multilayer absorbent panel (9);
- turning up said first pair of free edges (21) of said web (20) of liquid-impermeable material on said effective areas (23) occupied by said multilayer absorbent panels (9) to define first lateral closing means of said absorbent mat ( 100) against the leakage of liquids;
- providing strips (4, 5) of a liquid-impermeable material;
- applying said strips (4, 5) straddling at least a portion of said free transverse areas (24) and said effective areas (23) occupied by said multilayer absorbent panels (9), to obtain second lateral closing means against the leakage of liquids, arranged orthogonally to said first lateral closing means;
- transversely cutting said web (20) to obtain said disposable absorbent mat (100).

10. Process for manufacturing a disposable absorbent mat (100) according to claim 9, **characterized in that** it further comprises the steps of:
- providing alternative strips (10) of a liquid-impermeable material having a width greater than the width of said free transverse areas (24) along said direction of advancement (x) of said web (20);
- applying each of said alternative strips (10) between two consecutive multilayer absorbent panels (9);
- transversely cutting said web (20) at a median line of said alternative strips (10) to obtain said strips (4, 5).

11. Process for manufacturing a disposable absorbent mat (100) according to claim 9 or 10, **characterized in that** said steps of coupling together said absorbent pads (1) and said sheets of liquid-permeable material (3), of applying said multilayer absorbent panels (9) on top of said web (20) of liquid-impermeable material, and of applying said strips (4, 5, 10), comprise the steps of gluing or welding said components together.

## Patentansprüche

1. Einweg-Absorptionsmatte (100) mit im Wesentlichen viereckiger Form, folgendes umfassend:
- ein Flüssigkeitsabsorptionskissen (1) mit einer vorgegebenen Länge und Breite, das eine Wirkfläche (23) begrenzt;
- eine Folie aus flüssigkeitsundurchlässigem Material (2) mit einer vorbestimmten Länge und Breite, die größer sind als die Länge und Breite des besagten Absorptionskissen (1), die unter dem besagten Absorptionskissen (1) gelegt verbunden ist, so dass ein erstes (21) und ein zweites (22) Paar gegenüberliegender freier Kanten gebildet wird, die außerhalb der vom besagten Absorptionskissen (1) eingenommenen Wirkfläche (23) angeordnet sind;
- eine Folie aus flüssigkeitsdurchlässigem Material (3), die auf der der Folie aus flüssigkeitsundurchlässigem Material (2) gegenüberliegenden Seite auf dem besagten Absorptionskissen (1) aufgelegt verbunden ist,
wobei das besagte erste Paar freier Kanten (21) der besagten Folie aus flüssigkeitsundurchlässigem Material (2) auf die von dem besagten Absorptionskissen (1) eingenommenen besagte Wirkfläche (23) umgeschlagen ist, um eine erste seitliche Verschlussvorrichtung des besagten Absorptionskissens (1) gegen das Austreten von Flüssigkeiten zu bilden,
**dadurch gekennzeichnet, dass** die besagte Folie aus flüssigkeitsdurchlässigem Material (3) eine vordefinierte Länge und Breite aufweist, die der Länge und Breite des besagten Absorptionskissens (1) entsprechen, und dass die besagte Absorptionsmatte (100) einen ersten (4) und zweiten (5) Streifen aus flüssigkeitsundurchlässigem Material umfasst, der jeweils über jede freie Kante des besagten zweiten Paares freier Kanten (22) und der besagten Folie aus flüssigkeitsdurchlässigem Material (3) gelegt sind, wobei der besagte erste (4) und zweite (5) Streifen zumindest mit dem besagten zweiten Paar freier Kanten (22) verbunden sind, um eine zweite seitliche Verschlussvorrichtung des besagten Absorptionskissens (1) gegen das Austreten von Flüssigkeiten zu bilden, die orthogonal zu der besagten ersten seitlichen Verschlussvorrichtung angeordnet ist.

2. Einweg-Absorptionsmatte (100) gemäß Anspruch 1, wobei das besagte erste Paar freier Kanten (21) der besagten Folie aus flüssigkeitsundurchlässigem Material (2) auf das besagte flüssigkeitsdurchlässige Material (3) umgeschlagen ist.

3. Einweg-Absorptionsmatte (100) gemäß Anspruch 2, wobei der besagte erste (4) und zweite (5) Streifen auf die Enden der besagten ersten seitlichen Verschlussvorrichtung aufgelegt sind, die durch das erste Paar hochgeschlagener freier Kanten (21) gebildet wird.

4. Einweg-Absorptionsmatte (100) gemäß einem jeglichen der vorhergehenden Ansprüche, **dadurch gekennzeichnet, dass** der besagte erste (4) und zweite (5) Streifen aus undurchlässigem Material aus einem hydrophoben Nichtgewebe bestehen.

5. Einweg-Absorptionsmatte (100) gemäß einem jeglichen der vorhergehenden Ansprüche, **dadurch gekennzeichnet, dass** die besagte Folie aus flüssigkeitsundurchlässigem Material (2) Polyethylen enthält.

6. Einweg-Absorptionsmatte (100) gemäß einem jeglichen der vorhergehenden Ansprüche, **dadurch gekennzeichnet, dass** die besagte Folie aus flüssigkeitsdurchlässigem Material (3) aus einem hydrophilen Nichtgewebe besteht.

7. Einweg-Absorptionsmatte (100) gemäß einem jeglichen der vorhergehenden Ansprüche, **dadurch gekennzeichnet, dass** das besagte Absorptionskissen (1) aus mehreren Schichten besteht und mindestens ein Gewebe (6) aus hydrophilem Material und eine Schicht (7) aus absorbierender Zellulose umfasst, wobei das besagte mindestens eine Gewebe (6) der besagten Folie aus flüssigkeitsundurchlässigem Material (2) zugewandt ist und die besagte Schicht (7) aus absorbierender Zellulose der besagten Folie aus flüssigkeitsdurchlässigem Material (3) zugewandt ist.

8. Einweg-Absorptionsmatte (100) gemäß einem jeglichen der vorhergehenden Ansprüche, **dadurch gekennzeichnet, dass** das besagte Absorptionskissen (1), die besagte Folie aus flüssigkeitsundurchlässigem Material (2), die besagte Folie aus flüssigkeitsdurchlässigem Material (3), die besagten Paare von Kanten (21, 22) und die besagten Streifen (4, 5) durch Klebstoff (8) oder Verschweißen miteinander verbunden sind.

9. Verfahren zur Herstellung einer Einweg-Absorptionsmatte (100) gemäß einem jeglichen der Ansprüche 1-8, **dadurch gekennzeichnet, dass** es die folgenden Schritte umfasst:
- Bereitstellen einer Bahn (20) aus flüssigkeitsundurchlässigem Material mit einer vorgegebenen Breite und Abwickeln derselben in einer Vorschubrichtung (x);
- Bereitstellen einer Vielzahl von Flüssigkeitsabsorptionskissen (1), die jeweils eine vordefinierte Länge und Breite aufweisen, wobei die besagte Breite geringer ist als die Breite der besagten Bahn (20);
- Bereitstellen einer Vielzahl von Folien aus flüssigkeitsdurchlässigem Material (3), die jeweils eine vordefinierte Länge und Breite aufweisen, die der Länge und Breite der besagten Absorptionskissen (1) entsprechen;
- das Zusammenfügen der besagten Absorptionskissen (1) und der besagten Folien aus flüssigkeitsdurchlässigem Material (3) zur Bildung mehrschichtiger Absorptionspanele (9);
- Anbringen der besagten mehrschichtigen Absorptionspanele (9) auf die besagte Bahn (20) aus flüssigkeitsundurchlässigem Material gemäß der besagten Vorschubrichtung (x), sequenziell und im Abstand voneinander, um entlang der besagten Bahn (20) Wirkflächen (23) und ein erstes Paar gegenüberliegender freier Kanten (21) entlang der besagten Vorschubrichtung (x) und freie Bereiche (24) zwischen jedem mehrschichtigen Absorptionspanel (9) quer zur besagten Vorschubrichtung (x) zu bilden;
- Umschlagen des besagten ersten Paares freier Kanten (21) der besagten Bahn (20) aus flüssigkeitsundurchlässigem Material über die von den mehrschichtigen Absorptionspanelen (9) eingenommenen Wirkflächen (23), um erste seitliche Verschlussvorrichtungen der besagten Absorptionsmatte (100) gegen das Austreten von Flüssigkeiten zu bilden;
- Bereitstellen von Streifen (4, 5) aus flüssigkeitsundurchlässigem Material;
- Anbringen der besagten Streifen (4, 5) so, dass sie zumindest einen Teil der besagten freien Querbereiche (24) und der besagten Wirkflächen (23) überspannen, die von den besagten mehrschichtigen Absorptionspanelen (9) eingenommen werden, um zweite seitliche Verschlussvorrichtungen gegen das Austreten von Flüssigkeiten zu erhalten, die orthogonal zu den besagten ersten seitlichen Verschlussvorrichtungen angeordnet ist;
- quer Zuschneiden der besagten Bahn (20), um die besagte Einweg-Absorptionsmatte (100) zu erhalten.

10. Verfahren zur Herstellung einer Einweg-Absorptionsmatte (100) gemäß Anspruch 9, **dadurch gekennzeichnet, dass** es ferner die folgenden Schritte umfasst:
- Bereitstellung alternativer Streifen (10) aus einem flüssigkeitsundurchlässigen Material, deren Breite größer ist als die Breite der besagten freien Querbereiche (24) entlang der besagten Vorschubrichtung (x) der besagten Bahn (20);
- Anbringen jedes der besagten alternativen Streifen (10) zwischen zwei aufeinanderfolgenden mehrschichtigen Absorptionspanele (9);
- quer Zuschneiden der besagten Bahn (20) an einer Mittellinie der besagten alternativen Streifen (10), um die besagten Streifen (4, 5) zu erhalten.

11. Verfahren zur Herstellung einer Einweg-Absorptionsmatte (100) gemäß Anspruch 9 oder 10, **dadurch gekennzeichnet, dass** die besagten Schritte des Zusammenfügens der besagten Absorptionskissen (1) und der besagten Folien aus flüssigkeitsdurchlässigem Material (3), des Anbringens der besagten mehrschichtigen Absorptionspanele (9) auf die besagte Bahn (20) aus flüssigkeitsundurchlässigem Material, sowie des Anbringens der besagten Streifen (4, 5, 10) die Schritte des Zusammenklebens oder -verschweißens der besagten Komponenten umfassen.

## Revendications

1. Tapis absorbant jetable (100), de forme substantiellement quadrangulaire, comprenant :
- un tampon d'absorption de liquide (1) ayant une longueur et une largeur prédéfinies délimitant une zone efficace (23) ;
- une feuille de matériau imperméable aux liquides (2), ayant une longueur et une largeur prédéfinies supérieures à la longueur et à la largeur dudit tampon absorbant (1), couplée disposée sous ledit tampon absorbant (1), de manière à définir une première (21) et une seconde (22) paire de bords libres opposés, disposées à l'extérieur de la zone efficace (23) occupée par ledit tampon absorbant (1) ;
- une feuille de matériau perméable aux liquides (3), couplée superposée audit tampon absorbant (1) du côté opposé par rapport à ladite feuille de matériau imperméable aux liquides (2),
où ladite première paire de bords libres (21) de ladite feuille de matériau imperméable aux liquides (2) est rabattue sur ladite zone efficace (23) occupée par ledit tampon absorbant (1) pour définir des premiers moyens de fermeture latérale dudit tampon absorbant (1) contre la fuite de liquides,
**caractérisé par le fait que** ladite feuille de matériau perméable aux liquides (3) a une longueur et une largeur prédéfinies égales à la longueur et à la largeur dudit tampon absorbant (1), et **par le fait que** ledit tapis absorbant (100) comprend une première (4) et une seconde (5) bande de matériau imperméable aux liquides placées respectivement à cheval sur chaque bord libre de ladite seconde paire de bords libres (22) et ladite feuille de matériau perméable aux liquides (3), dans laquelle lesdites première (4) et seconde (5) bandes sont couplées au moins à ladite seconde paire de bords libres (22) pour définir des seconds moyens de fermeture latérale dudit tampon absorbant (1) contre la fuite de liquides, agencées orthogonalement par rapport auxdits premiers moyens de fermeture latérale.

2. Tapis absorbant jetable (100) selon la revendication 1, où ladite première paire de bords libres (21) de ladite feuille de matériau imperméable aux liquides (2) est rabattue sur ledit matériau perméable aux liquides (3).

3. Tapis absorbant jetable (100) selon la revendication 2, où lesdites première (4) et seconde (5) bandes sont superposées aux extrémités desdits premiers moyens de fermeture latérale formés par la première paire de bords libres (21) qui ont été rabattus.

4. Tapis absorbant jetable (100) selon l'une des revendications précédentes, **caractérisé par le fait que** lesdites première (4) et seconde (5) bandes de matériau imperméable comprennent un tissu non tissé hydrophobe.

5. Tapis absorbant jetable (100) selon l'une des revendications précédentes, **caractérisé par le fait que** ladite feuille de matériau imperméable aux liquides (2) comprend du polyéthylène.

6. Tapis absorbant jetable (100) selon l'une des revendications précédentes, **caractérisé par le fait que** ladite feuille de matériau perméable aux liquides (3) comprend un tissu non tissé hydrophile.

7. Tapis absorbant jetable (100) selon l'une des revendications précédentes, **caractérisé par le fait que** ledit tampon absorbant (1) est composite, et comprend au moins un tissu (6) en matériau hydrophile et une couche (7) de cellulose absorbante, dans laquelle ledit au moins un tissu (6) est en regard de ladite feuille de matériau imperméable aux liquides (2) et ladite couche de cellulose absorbante (7) est en regard de ladite feuille de matériau perméable aux liquides (3).

8. Tapis absorbant jetable (100) selon l'une des revendications précédentes, **caractérisé par le fait que** ledit tampon absorbant (1), ladite feuille de matériau imperméable aux liquides (2), ladite feuille de matériau perméable aux liquides (3), lesdites paires de bords (21, 22) et lesdites bandes (4, 5) sont couplés ensemble par collage (8) ou soudage.

9. Procédé de fabrication d'un tapis absorbant jetable (100) selon l'une des revendications 1 à 8, **caractérisé par le fait qu'**il comprend les étapes suivantes :
- fournir une nappe (20) de matériau imperméable aux liquides ayant une largeur prédéfinie et la dérouler selon une direction d'avancement (x) ;
- fournir une multitude de tampons d'absorption de liquide (1), ayant chacun une longueur et une largeur prédéfinies, ladite largeur étant inférieure à la largeur de ladite nappe (20) ;
- fournir une multitude de feuilles de matériau perméable aux liquides (3), ayant chacune une longueur et une largeur prédéfinies égales à la longueur et à la largeur desdits tampons absorbants (1) ;
- coupler ensemble lesdits tampons absorbants (1) et lesdites feuilles de matériau perméable aux liquides (3) pour créer des panneaux absorbants multicouches (9) ;
- appliquer lesdits panneaux absorbants multicouches (9) sur le dessus de ladite nappe (20) de matériau imperméable aux liquides selon ladite direction d'avancement (x), en séquence et espacés, de manière à définir, le long de ladite nappe (20), des zones efficaces (23) et une première paire de bords libres opposés (21) selon ladite direction d'avancement (x) et des zones libres transversales (24) par rapport à ladite direction d'avancement (x) entre chaque panneau absorbant multicouche (9) ;
- rabattre ladite première paire de bords libres (21) de ladite nappe (20) de matériau imperméable aux liquides sur lesdites zones efficaces (23) occupées par lesdits panneaux absorbants multicouches (9) pour définir des premiers moyens de fermeture latérale dudit tapis absorbant (100) contre la fuite de liquides;
- fournir des bandes (4, 5) d'un matériau imperméable aux liquides ;
- appliquer lesdites bandes (4, 5) à cheval sur au moins une partie desdites zones libres transversales (24) et desdites zones efficaces (23) occupées par lesdits panneaux absorbants multicouches (9), pour obtenir des seconds moyens de fermeture latérale contre la fuite de liquides, agencés orthogonalement auxdits premiers moyens de fermeture latérale ;
- couper transversalement ladite nappe (20) pour obtenir ledit tapis absorbant jetable (100).

10. Procédé de fabrication d'un tapis absorbant jetable (100) selon la revendication 9, **caractérisé par le fait qu'**il comprend en outre les étapes suivantes :
- fournir des bandes alternatives (10) d'un matériau imperméable aux liquides ayant une largeur supérieure à la largeur desdites zones libres transversales (24) selon ladite direction d'avancement (x) de ladite nappe (20) ;
- appliquer chacune desdites bandes alternatives (10) entre deux panneaux absorbants multicouches (9) consécutifs (9) ;
- couper transversalement ladite nappe (20) selon une ligne médiane desdites bandes alternatives (10) pour obtenir lesdites bandes (4, 5).

11. Procédé de fabrication d'un tapis absorbant jetable (100) selon la revendication 9 ou 10, **caractérisé par le fait que** lesdites étapes de couplage desdits tampons absorbants (1) et desdites feuilles de matériau perméable aux liquides (3), d'application desdits panneaux absorbants multicouches (9) sur le dessus de ladite nappe (20) de matériau imperméable aux liquides, et d'application desdites bandes (4, 5, 10), comprennent les étapes de collage ou de soudage desdits composants ensemble.
